# EUROPÄISCHE PATENTANMELDUNG

(11) **EP 1 421 853 A1**
(43) Veröffentlichungstag der Anmeldung: **26.05.2004**
(21) Anmeldenummer: 02026138.4
(22) Anmeldetag: 23.11.2002
(51) Int. Cl.: A01N 61/00, A01N 43/16, A61K 7/40

(54) **Insektenabwehrmittel**

(71) Anmelder: Beiersdorf AG, 20245 Hamburg (DE)
(72) Erfinder: Lanzendörfer, Ghita, Dr., 22087 Hamburg (DE); Wolf, Florian, Dr., 37671 Höxter (DE); Kröpke, Rainer, 22869 Schenefeld (DE); Von Thaden, Stefanie, 20255 Hamburg (DE); Sauermann, Gerhard, Dr., 15324 Neuendorf (DE)

(57) **Zusammenfassung**

Wirkstoffkombinationen zur Abwehr und/oder Vertreibung von stechenden oder beißenden Insekten und/oder anderen Parasiten und/oder Lästlingen aus mindestens einem Repellent-Wirkstoff und mindestens einem antimikrobiellen Wirkstoff.

## Beschreibung

Die vorliegende Erfindung betrifft Insektenabwehrmittel, bevorzugt topische, kosmetische oder dermatologische Zubereitungen zur gezielten Verhinderung von Insektenstichen.

Insektenabwehrmittel (Insektenvertreibungsmittel, Insektenschutzmittel, Repellentien, Repellents) sind Präparate, die zur Abwehr und/oder Vertreibung von Insekten, aber auch Zecken und Milben äußerlich angewendet werden und verhindern sollen, daß diese auf der Haut aktiv werden. Insektenabwehrmittel sollen die Haut vor Belästigung durch blutsaugende oder beißende Insekten und andere Parasiten und/oder Lästlinge schützen, indem sie diese abwehren, bevor sie auf die Haut fliegen, so daß es nicht zu Stichen oder Bissen kommt. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenabwehrmittel" nicht nur solche Formulierungen zu verstehen, die gegen Insekten wirksam sind. Vielmehr gilt das nachstehend Gesagte selbstverständlich auch für solche Präparate, die andere blutsaugende oder beißende Parasiten und/oder Lästlinge abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitteln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs überwiegend unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hochsiedende Flüssigkeiten oder niedrig schmelzende kristalline Stoffe, die bei Raumtemperatur langsam verdampfen. Die meisten Repellent-Wirkstoffe gehören den Stoffklassen der Amide, Alkohole, Ester und Ether an.

Repellent-Wirkstoffe sollen die folgenden Bedingungen erfüllen:

Sie dürfen nicht zu schnell verdunsten und nicht in die Haut eindringen. Sie dürfen auf die Haut weder primär irritierend noch sensibilisierend wirken und sollen außerdem nicht toxisch sein. Ihre Wirksamkeit muß auch unter Einwirkung von Hautflüssigkeit und/oder UV-Strahlung erhalten bleiben.

Von den heute in Insektenabwehrmitteln ca. 15 häufig eingesetzten Wirkstoffen wird das N,N-Diethyl-3-methylbenzamid (DEET), welches sich durch die folgende Strukturformel auszeichnet 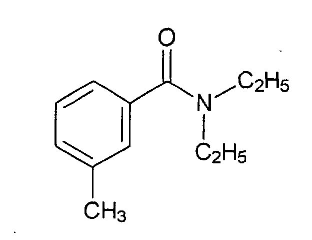 als bestes Allround-Repellent bezeichnet. Es wirkt abwehrend gegen Stechmücken, Bremsen, Sandfliegen, Zecken, Stechfliegen, Milben, Flöhe und Wanzen, wobei die Wirkungsdauer - wie bei allen Repellent-Wirkstoffen - unterschiedlich lang gegenüber den verschiedenen Spezies ist. Handelsübliche DEET-Präparate beispielsweise sind ca. 6 bis 8 Stunden gegen Mücken wirksam, jedoch nur ca. 2 bis 4 Stunden gegen Zecken.

Ein weiterer gebräuchlicher Repellent-Wirkstoff ist der 3-(N-n-Butyl-N-acetyl-amino)propionsäureethylester (auch als Repellent 3535 bezeichnet), welcher sich durch die folgende Strukturformel auszeichnet

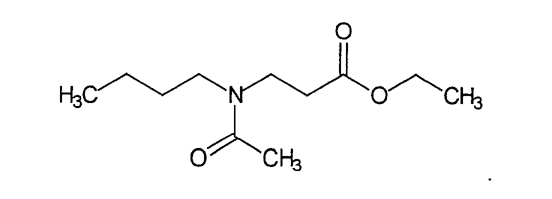

Repellent 3535 ist gegen Stechmücken (Aedes aegypti, Anopheles albimanus), Tsetsefliegen (Glossinae) und Bremsen (Tabanidae) wirksam.

Ferner gebräuchlich ist Dimethylphthalat (Palatinol M, DMP) 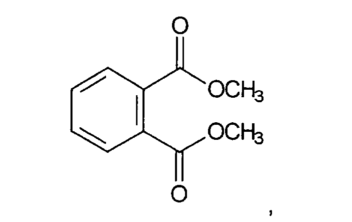 welches gegen Stechmücken (insbesondere Aedes- und Anopheles-Arten), Läuse, Zecken und Milben wirksam ist, allerdings vorwiegend in Kombination mit weiteren Repellent-Wirkstoffen eingesetzt wird.

Insektenabwehrmittel werden in Form von Lösungen, Emulsionen, Gelen, Stiften, Rollem, Pump-Sprays und Aerosol-Sprays angeboten, wobei Lösungen und Sprays den Hauptteil der im Handel erhältlichen Produkte bilden. Basis für beide Produktformen sind meist alkoholische bzw. wäßrig-alkoholische Lösungen unter Zusatz fettender Substanzen und leichter Parfümierungen. Die Wirkungsdauer der Mittel nimmt üblicherweise mit der Konzentration des insektenabwehrenden Wirkstoffes im Fertigprodukt zu, welche in der Regel zwischen 20 und 70 Gew.-% beträgt. Sie ist ferner von der Schichtdicke beim Auftragen abhängig sowie von der Intensität der Schweißabsonderung und der Außentemperatur.

Stiche oder Bisse von Insekten und anderen Parasiten führen normalerweise allenfalls zu Quaddelbildung, Rötung und Juckreiz sowie in vereinzelten Fällen zu meist harmlos verlaufenden Infektionen. Insekten, insbesondere Mücken können aber auch Überträger parasitärer und viraler Infektionen (wie z. B. Malaria, Gelbfieber oder Dengue-Fieber) sein. Insgesamt gibt es z. B. nicht weniger als 3000 verschiedene Stechmückenarten, von denen etwa 100 Seuchen verbreiten können. Die Abwehr oder Vertreibung dieser Insekten dient daher insbesondere auch dem Schutz vor solchen Infektionen.

An sich ist die Verwendung der üblichen Repellent-Wirkstoffe in kosmetischen oder dermatologischen Zubereitungen unbedenklich. Dennoch können Repellent-Wirkstoffe, wie letztlich jede chemische Substanz, im Einzelfalle allergische oder auf Überempfindlichkeit des Anwenders beruhende Reaktionen hervorrufen, weshalb ihre Konzentration in Insektenabwehrmitteln möglichst niedrig gehalten werden sollte. Als unerwünschte Nebenwirkungen können bei empfindlichen Personen beispielsweise Hautreizungen auftreten. Ferner können Insektenabwehrmittel auf den Schleimhäuten oder auf wunder Haut Brennen und Schmerz hervorrufen. Bei unsachgemäßer Anwendung besteht ferner die Gefahr von Augenreizungen.

Demgegenüber steht, daß die abzuschreckenden Insekten inzwischen vielfach resistent gegenüber den üblichen Repellent-Wirkstoffen geworden sind und somit nicht mehr erfolgreich bekämpft werden können. Damit einhergehend ist sowohl für die Bewohner entsprechender Gebiete als auch für Reisende zusehends weniger prophylaktischer Schutz vor Infektionen möglich.

Aufgabe der vorliegenden Erfindung war es daher, den Nachteilen des Standes der Technik abzuhelfen und kosmetische oder dermatologische Zubereitungen zur Insektenabwehr zu finden, welche sich durch eine hohe Wirksamkeit auszeichnen und in welchen dennoch die Einsatzmenge herkömmlicher Repellent-Wirkstoffe auf ein Minimum reduziert werden kann.

Die Wirkungsweise von Repellent-Wirkstoffen stellt man sich so vor, daß diese nach der Applikation langsam verdampfen und dadurch dicht über der Haut einen Duftmantel bilden, der auf die Insekten abstoßend wirkt, indem die Wirkstoffe auf die für den "Geruchssinn" verantwortlichen Sensoren der Insekten ansprechen. Sie greifen damit störend in den Lockmechanismus ein. Nach dem Auftragen eines Insektenabwehrmittels fliegen die Insekten bereits in einiger Entfernung von der Haut wieder ab; sie machen sozusagen "einen Bogen" um die behandelten Hautpartien. Mit nachlassender Wirkung nähern sie sich mehr und mehr, bis schließlich die Konzentration der Repellents so weit abgesunken ist, daß der erste Kontakt bzw. Stich stattfindet.

Insekten orientieren sich optisch und mit Hilfe "chemischer Sinne" nach Licht, Form und Farbe, Wärme und Luftfeuchtigkeit sowie dem Gehalt der Luft an Kohlendioxid und Duftstoffen. Hierzu gehören verschiedene Aminosäuren sowie Ammoniak, Milch- und Buttersäure. Sie "erkennen" für sie vielversprechende Wirtsorganismen insbesondere an deren Körpertemperatur, dem Kohlendioxidausstoß und der durch die kutane Mikroflora erzeugten und emittierten Geruchsstoffe. Die Lockstoffe werden in Richtung zunehmender Konzentration aufgesucht (*P. Finkel, E. Siemer, "Repellents zur dermalen Anwendung", Apoth.J.* ***8****, 1986, S. 32-37*).

Die Körpertemperatur und der Kohlendioxidausstoß eines Menschen lassen sich durch topisch applizierbare Formulierungen - wenn überhaupt - nur unzureichend beeinflussen. Die Wirkungsweise von Insektenabwehrmitteln beschränkt sich daher im wesentlichen darauf, emittierte Geruchsstoffe zu überdecken bzw. ihren Ausstoß oder besonders vorteilhaft ihre Produktion zu vermindern bzw. gänzlich zu unterdrücken.

Der Einsatz von antimikrobiellen Wirkstoffen in Kosmetika und Dermatika ist an sich selbstverständlich bekannt. Antimikrobielle Wirkstoffe werden in großer Zahl und Vielfalt in kosmetischen oder dermatologischen Desodorantien eingesetzt, um unangenehmem Körpergeruch entgegenzuwirken. Körpergeruch entsteht, wenn der an sich geruchlose frische Schweiß durch Mikroorganismen zersetzt wird. Durch die Verwendung antimikrobieller Wirkstoffe in Desodorantien kann die Bakterienflora auf der Haut reduziert und so die mikrobielle Zersetzung des frischen Schweißes verhindern werden.

Antimikrobielle Wirkstoffe haben idealerweise eine weitgehend selektive Wirksamkeit gegenüber den für die Entstehung des Körpergeruchs verantwortlichen Bakterien. Durch ihre Anwendung soll die Population der betroffenen Bakterien lediglich kontrolliert und ein zu starkes Wachstum verhindert werden (bakteriostatische Wirkung). Keinesfalls hingegen soll die Population vollständig abgetötet werden (keine bakterizide Wirkung), da in diesem Fall das biologische Gleichgewicht der Hautflora stark beeinträchtigt würde, so daß sich Krankheitserreger ausbreiten könnten. Naturgemäß gibt es eine ganze Reihe von Wirkstoffen mit keimhemmender Wirkung, die die genannten Kriterien mehr oder weniger gut erfüllen. Demensprechend sind diverse antimikrobielle Wirkstoffe handelsübliche Produkte.

Allerdings konnte der Stand der Technik keinerlei Hinweise geben, welche Rückschlüsse auf die erfindungsgemäßen Zubereitungen erlaubt hätten.

Es war erstaunlich und für den Fachmann in keiner Weise vorhersehbar, daß
Wirkstoffkombinationen zur Abwehr und/oder Vertreibung von stechenden oder beißenden Insekten und/oder anderen Parasiten und/oder Lästlingen aus mindestens einem Repellent-Wirkstoff und mindestens einem antimikrobiellen Wirkstoff
den Nachteilen des Standes der Technik abhelfen.

Es hat sich in erstaunlicher Weise herausgestellt, daß die erfindungsgemäßen Wirkstoffkombinationen die Haut vor Belästigung durch blutsaugende oder beißende Insekten und andere Parasiten und/oder Lästlingen schützen, indem sie diese abwehren, bevor sie auf die Haut fliegen. Dabei wirken die erfindungsgemäßen Wirkstoffkombinationen in synergistischer Weise, also überadditiv in bezug auf die Einzelkomponenten. Dies zeigt sich beispielsweise darin, daß die Schutzleistung dieser Zubereitungen höher ist als die gleicher Zubereitungen, welche keinen antimikrobiellen Wirkstoff enthalten.

Vorteilhafte antimikrobielle Wirkstoffe bzw. Wirksysteme im Sinne der vorliegenden Erfindung sind:
• unverzweigte und/oder ein- oder mehrfach verzweigte, gesättigte und/oder ungesättigte Fettalkohole, Fettaldehyde und Fettsäuren mit bis zu fünf Doppel- und/oder Dreifachbindungen und einer Kettenlänge von 2 bis 40 Kohlenstoffatomen,
• Aryl- oder Aryloxy-substituierte unverzweigte und/oder ein- oder mehrfach verzweigte, gesättigte und/oder ungesättigte Fettalkohole, Fettaldehyde und Fettsäuren mit bis zu fünf Doppel- und/oder Dreifachbindungen und einer Kettenlänge von 2 bis 40 Kohlenstoffatomen,
• Mono- und Oligoglyceride mit bis zu 4 Glycerin-Einheiten von unverzweigten und/oder ein- oder mehrfach verzweigten, gesättigten und/oder ungesättigten Fettalkoholen (Mono- bzw. Oligoglycerinmonoalkylether) und Fettsäuren (Mono- und Oligoglycerinmonoalkylester) mit bis zu fünf Doppel- und/oder Dreifachbindungen und einer Kettenlänge von 2 bis 40 Kohlenstoffatomen,
• Mono- und Oligoglyceride mit bis zu 4 Glycerin-Einheiten von Aryl- oder Aryloxy-substituierten, unverzweigten und/oder ein- oder mehrfach verzweigten, gesättigten und/oder ungesättigten Fettalkoholen (Mono- bzw. Oligoglycerinmonoalkylether) und Fettsäuren (Mono- und Oligoglycerinmonoalkylester) mit bis zu fünf Doppel- und/oder Dreifachbindungen und einer Kettenlänge von 2 bis 40 Kohlenstoffatomen,
• pflanzliche und tierische Fettsäureschnitte, enthaltend unverzweigte und/oder einoder mehrfach verzweigte, gesättigte und/oder ungesättigte Fettalkohole, Fettaldehyde und Fettsäuren mit bis zu fünf Doppel- und/oder Dreifachbindungen und einer Kettenlänge von 2 bis 40 Kohlenstoffatomen, wie z. B. Kokosfettsäuren, Palmkernfettsäuren, Wollwachssäuren),
• unverzweigte und/oder ein- oder mehrfach verzweigte, gesättigte und/oder ungesättigte Alkandiole, Dialdehyde und/oder Dicarbonsäuren mit bis zu fünf Doppel- und/oder Dreifachbindungen und einer Kettenlänge von 2 bis 40 Kohlenstoffatomen,
• Aryl- oder Aryloxy-substituierte unverzweigte und/oder ein- oder mehrfach verzweigte, gesättigte und/oder ungesättigte Alkandiole, Dialdehyde und/oder Dicarbonsäuren mit bis zu fünf Doppel- und/oder Dreifachbindungen und einer Kettenlänge von 2 bis 40 Kohlenstoffatomen,
• Mono- und Oligoglyceride mit bis zu 4 Glycerin-Einheiten von unverzweigten und/oder ein- oder mehrfach verzweigten, gesättigten und/oder ungesättigten Alkandiolen (Mono- und Oligoglycerinmonoalkylether; Bis[mono-/oligoglyceryl]alkyldiether) und Dicarbonsäuren (Mono- und Oligoglycerinmonoalkylester; Bis[mono-/oligoglyceryl]alkyldiester) mit bis zu fünf Doppel- und/oder Dreifachbindungen und einer Kettenlänge von 2 bis 40 Kohlenstoffatomen,
• Mono- und Oligoglyceride mit bis zu 4 Glycerin-Einheiten von Aryl- oder Aryloxy-substituierten unverzweigten und/oder ein- oder mehrfach verzweigten, gesättigten und/oder ungesättigten Alkandiolen (Mono- und Oligoglycerinmonoalkylether; Bis[mono-/oligoglyceryl]alkyldiether) und Dicarbonsäuren (Mono- und Oligoglycerinmonoalkylester; Bis[mono-/oligoglyceryl]alkyldiester) mit bis zu fünf Doppel- und/oder Dreifachbindungen und einer Kettenlänge von 2 bis 40 Kohlenstoffatomen,
• pflanzliche und tierische Fettsäureschnitte, enthaltend unverzweigte und/oder ein- oder mehrfach verzweigte, gesättigte und/oder ungesättigte Alkandiole, Dialdehyde und/oder Dicarbonsäuren mit bis zu fünf Doppel- und/oder Dreifachbindungen und einer Kettenlänge von 2 bis 40 Kohlenstoffatomen,
• Fettsäureester von unverzweigten und/oder ein- oder mehrfach verzweigten, gesättigten und/oder ungesättigten, gegebenenfalls auch Aryl- oder Aryloxy-substituierten Carbonsäuren mit bis zu fünf Doppel- und/oder Dreifachbindungen und einer Kettenlänge von 2 bis 40 Kohlenstoffatomen mit unverzweigten und/oder ein- oder mehrfach verzweigten, gesättigten und/oder ungesättigten, gegebenenfalls auch Aryl- oder Aryloxy-substituierten ein- bis sechswertigen Fettalkoholen mit bis zu fünf Doppel- und/oder Dreifachbindungen und einer Kettenlänge von 2 bis 40 Kohlenstoffatomen ,
• ein- und/oder mehrfach halogenierte Nitrile, Dinitrile, Trinitrile oder Tetranitrile gewählt aus der Gruppe der Verbindungen, welche sich durch die Strukturformel 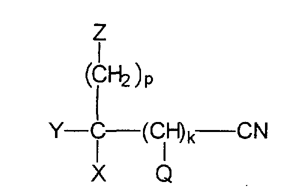 auszeichnen, worin
Q ein Halogen- oder Wasserstoffatom darstellt,
X, Y und Z unabhängig voneinander gewählt werden aus der Gruppe Halogenatom, Wasserstoffatom, -CN und verzweigte oder unverzweigte Methyl- bis Octadecyl-Reste und
p zwischen 0 und 10 und k zwischen 0 und 18 gewählt werden,
• Arylverbindungen gewählt aus der Gruppe der Verbindungen, welche sich durch die Strukturformel: 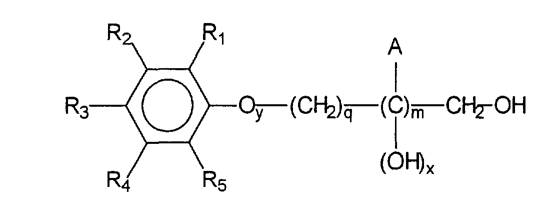 auszeichnen, worin
R₁ bis R₅ unabhängig voneinander gewählt werden aus der Gruppe H, Methyl-, Methoxy- und Amino-,
A gewählt wird aus der Gruppe H, Methyl-, Ethyl- und
q = zwischen 0 und 10, m zwischen 0 und 10, y als 0 oder 1 und x als 0 oder 1 gewählt werden, wobei, sofern m größer 1 gewählt wird, innerhalb eines Moleküls x für alle Kohlenstoffatome identische oder unterschiedliche Werte annehmen kann.
• Mono- und Oligohydroxyfettsäuren der Kettenlängen C₂ bis C₂₄ (z. B. Milchsäure, 2-Hydroxypalmitinsäure), deren Oligo- und/oder Polymere sowie pflanzliche und tierische Rohstoffe, dieselben enthaltend,
• unsubstituierte und alkylsubstituierte Hydrochinone sowie Pflanzenextrakte, dieselben enthaltend (z. B. Salbeiextrakt, Rosmarinextrakt),
• Terpene:
Acyclische Terpene wie Terpenkohlenwasserstoffe (z. B. Ocimen, Myrcen), Terpenalkohole (z. B. Geraniol, Linalool, Citronellol), Terpenaldehyde und -ketone (z. B. Citral, Pseudoionon, β-Ionon), monozyklische Terpene wie Terpenkohlenwasserstoffe (z. B. Terpinen, Terpinolen, Limonen), Terpenalkohole (z. B. Terpineol, Thymol, Methol) und Terpenketone (z. B. Pulegon, Carvon), bizyklische Terpene wie Terpenkohlenwasserstoffe (z. B. Caran, Pinan, Bornan), Terpenalkohole (z. B. Borneol, Isoborneol) und Terpenketone (z. B. Campher), Sesquiterpene wie acyclische Sesquiterpene (z. B. Farnesol, Nerolidol), monocyclische Sesquiterpene (z. B. Bisabolol), bicyclische Sesquiterpene (z. B. Cadinen, Selinen Vetivazulen, Guajazulen), tricyclische Sesquiterpene (z. B. Santalen), Diterpene (z. B. Phytol), tricyclische Diterpene (z. B. Abietinsäure), Triterpene (Squalenoide; z. B. Squalen) und Tetraterpene,
• halogenierte aromatische Verbindungen (z. B. Trichlorocarbanilid, Hexachlorophen, Triclosan, Dichlorphenylether etc.),
• klassische Konservierungsmittel (z. B. Formaldehyd, Glutardialdehyd, Mezetroniumethylsulfat, Parabene (z. B. Methyl-, Ethyl-, Propyl- und Butylparaben), Sorbit, Dibromdicyanobutan, Imidazolidinylharnstoffe ("Germall"), Diazolidinylharnstoff, Isothiazolinone ("Kathon"), Methylchlorthiazolidin, Methylthiazolidin, organische Säuren (z. B. Benzoesäure, Sorbinsäure, Salicylsäure) sowie deren Ester, Glycole (z. B. Propylenglycol, 1,2-Dihydroxyalkane), pflanzliche Konservierungshelfer und Flavonoide (z. B. Lantadin A, Caryophyllen, Hesperidin, Diosmin, Phellandren, Apigenin, Quercetin, Hypericin, Aucubin, Diosgenin, Plumbagin, Corlilagin etc.) sowie deren glycosylierte Derivate (z. B. Glycosylrutin),
• ethoxylierte, propoxylierte oder gemischt ethoxylierte/propoxylierte Fettalkohole, Fettsäuren und Fettsäureester mit 2 bis 40 Kohlenstoffatomen und 1 bis 150 Ethoxy- (E/O-) und/oder Propoxy- (P/O-) Einheiten,
• antimikrobielle Peptide und Proteine mit einer Aminosäurezahl von 4 bis 200, z. B. Magainine, Magainin-Amide, PGLa, PYLa, PGSa, Xenopsin, Xenopsin Precursor Fragments (XPFs), Caerulein, Caerulein Precursor Fragments (CPFs), Caeridine, Brevinine, Esculentine, Bombinine, Dermaseptine, Tachyplesine, Polyphemusine, Lantibiotika (z. B. Epidermin, Gallidermin, Nisin, Subtilin, Pep5, Pediocine, Plantaricine, Leucocine, Cinnamycin, Duramycin, Ancovenin, Colicine, Pyocine, Bacteriocine, Microcine, Lactococcine, Lactacine, Mersacidine, Actagardine, Lacticine, Streptococcine, Salivarine, Carnocine, Lactocine, Lanthiopeptine etc.), Skin Antimicrobial Peptides (SAPs), Lingual Antimicrobial Peptides (LAPs), humane β-Defensine (insbesondere h-BD1 und h-BD2), Tracheal Antimicrobial Peptide (TAPs), Defensine, Neutrophil-Peptide (z. B. NP-1 bis NP-5; HNP-1 bis HNP-4; GPNP; Cryptidine; RatNP-1 bis RatNP-4, Sapecine, Drosocine, Cecropine, Andropine, Attacine, Sarcotoxine, Diptericine, Coelopterine, Apidaecine, Abaecine, Hymenoptaecine, Melittine, Aedes *aegyptii*-Defensine, Cathepsin D, Azurocidine, Lactoferrine und deren Hydrolysate sowie daraus gewonnene Peptide, Bactericidal/Permeability Increasing Proteins (BPIs), Elastasen, Cationic Microbial Proteins (CAPs), Lysozym, Serprocidine, Myeloperoxidase, Indolicidine; Major Basic Proteins (MBPs), Eosinophil Cationic Proteins (ECPs); Bactenecine; Macrophage Cationic Peptides (MCPs), Histatine, Amoebapore, Thionine, Cysteinreiche antimikrobielle Peptide aus Pflanzen (z. B. Mj-AMPs, Ac-AMPs, Rs-AFPs, Rs-nsLTPs, Rs-2S) und deren synthetische Analoga enthaltend L- und/oder D-Aminosäuren (z. B. MSI-78),
• natürliche Ceramide und Sphingosine pflanzlichen und tierischen Ursprungs, pflanzliche und tierische Rohstoffe dieselben enthaltend, sowie synthetisch hergestellte Ceramide und Sphingosine (z. B. Acyl-Ceramide, Questamide oder Pseudoceramide), aber auch durch chemische oder biologische Transformation aus natürlichen oder synthetischen Ceramiden und Sphingosinen sowie geeigneten pflanzlichen und tierischen Rohstoffen hergestellte Produkte, beispielsweise die im Folgenden genannten Wirkstoffe, wobei die Liste der genannten Substanzen selbstverständlich nicht limitierend sein soll:
a) Sphingosin; N-Monoalkylierte Sphingosine; N,N-Dialkylierte Sphingosine; Sphingosin-1-Phosphat; Sphingosin-1-Sulfat; Psychosin (Sphingosin-β-D-Galactopyranosid); Sphingosylphosphorylcholin; Lysosulfatide (Sphingosylgalactosylsulfat; Lysocerebrosidsulfat); Lecithin; Sphingomyelin; Sphinganin,
b) humane Ceramide 1- 7, humane Ceramide A und B,
c) synthetische Analoga: Acyl-Ceramide, Questamide oder Pseudoceramide,
d) Globotriaosylceramid; Globotetraosyceramid, Globopentaosylceramid; Forssmann-Glycolipid und Analoga,
e) Glycocerebroside (z. B. Glucocerebroside, Galactocerebroside, Lactocerebroside), N-Alkanoylcerebroside (z. B. N-Palmitoylcerebrosid, N-Stearoylcerebrosid), N-Alkenoylcerebroside (z. B. N-Nervonoylcerebrosid, N-Oleoylcerebrosid), Cerebrosidsulfat,
f) Ganglioside (z. B. Typen 1 bis 5, Asialogangliosid GM1, Asialogangliosid GM2, Asialogangliosid GM3, Monosialogangliosid GM1, Monosialogangliosid GM2, Monosialogangliosid GM3, Disialogangliosid GD1a, Disialogangliosid GD1b, Disialogangliosid GD2, Disialogangliosid GD3, Trisialogangliosid GT1b, Tetrasialogangliosid GQ1b),
g) kosmetische Ceramide, z. B. Ceramides GSL, Sphingoceryl LS, Sphingoceryl-Wachs LS 2958 und Sphingosomes AL (alle von Laboratoire Serobiologiques), Sphingolipid CB-1 (von Nikko), Glycocer, Glycocer-HA und -HALA (alle von Intergen), Glycoderm, Liposomes CLR und Thiosome (alle von Kurt Richter), Glycosome (von Pentapharm), Phospholipids FPA und FPT (beide von Bioiberica) sowie
h) die in folgender Aufstellung genannten, im Handel erhältlichen kosmetischen Rohstoffe:

| Handelsname | Lieferant |
|---|---|
| AFR LS | Serobiologiques |
| Ceramides LS | Serobiologiques |
| Dermatein GSL | Hormel |
| Lipodermol | Serobiologiques |
| Sphingoceryl LS | Serobiologiques |
| Sphingoceryl LS Powder | Serobiologiques |
| Sphingoceryl Wax LS 2958 B | Serobiologiques |
| Sphingosomes AL | Serobiologiques |
| Sphingolipid CB-1 | Nikko |
| GlycoCer. | Intergen |
| Glycocer.HA | Intergen |
| GlycoCer.HALA | Intergen |
| Glycoderm | Kurt Richter |
| Glycosome | Pentapharm |
| Liposomes CLR | Kurt Richter |
| Phospholipids FPA | Bioiberica |
| Phospholipids FPT | Bioiberica |
| Thiosome | Kurt Richter |

wobei die unter a) bis h) genannten Wirkstoffe sowohl einzeln als auch in beliebigen Mischungen untereinander vorliegen können,
• natürliche Sterole und Sterol-Derivate pflanzlichen, pilzlichen und tierischen Ursprungs, pflanzliche, pilzliche und tierische Rohstoffe, dieselben oder Vorstufen dazu enthaltend, synthetische Sterole und Sterol-Derivate sowie semisynthetisch entweder durch biologische oder chemische Transformation natürlicher und synthetischer Substanzen erhaltene Sterole und Sterol-Derivate, beispielsweise die im Folgenden genannten Wirkstoffe, wobei die Liste der genannten Substanzen selbstverständlich nicht limitierend sein soll:
a) Gonan, Cholestan, Cholestanol, Ergosterol (aus Hefen und Pilzen), Stigmasterol (aus Sojabohnen), Gallensäuren (z. B. Cholansäure, Cholsäure, Desoxycholsäure, Chenodesoxycholsäure oder Lithocholsäure) sowie deren Salze,
b) Cholesterol und Cholesterylether und/oder -ester unverzweigter und/oder ein- oder mehrfach verzweigter, gesättigter und/oder ungesättigter Fettalkohole und Fettsäuren mit bis zu fünf Doppel- und/oder Dreifachbindungen und einer Kettenlänge von 2 bis 40 Kohlenstoffatomen (wie z. B. Cholesterylhydroxystearat der Fa. Nissin Oil Mills oder Cholesterylisostearat der Fa. Kao), ethoxylierte und propoxylierte Cholesterole (wie z. B. Choleth-20 der Fa. Nikko und Choleth-24 der Fa. Fanning); Dihydrocholesterol und seine Derivate (z. B. Dihydrocholesteryloctadecanoat, Dihydrocholeth-20 und -30, alle von Nikko),
c) Phytosterole (z. B. Soya Sterol) und Phytosteryl-Derivate (z. B. PEG-5-Soya Sterol, PEG-40-Soya Sterol, PEG-25-Phytosterol, Soya Sterol Esters) sowie Dihydrophytosterol und -Derivate (z. B. Dihydrophytosteryloctadecanoat von Nikko),
d) Lanosterol (Fa. Croda oder Fa. Nikko), Sitosterol, Koprosterol und deren Derivate (z. B. β-Sitosterylacetat, Fa. Variati; Dinatrium Sitostereth-14 Sulfosuccinat, Fa. Rewo),
e) Lanolin, Lanolinalkohol (z. B. Eucerit, Fa. Beiersdorf) und Lanolinsäure (z. B. Fa. Croda) sowie deren ethoxylierte und/oder propoxylierte Derivate (z. B. Laneth-5, Laneth-50, Laneth-10-acetat, alle Fa. Croda; PEG-75-Lanolin, Fa. Westbrook; PEG-100-Lanolin, Fa. Croda; PPG-5-Lanolin Wax, Fa. Henkel; PPG-12-PEG-50-Lanolin, PPG-12-PEG-65-Lanolin Oil, alle Fa. Croda; PPG-2-Lanolin. Alkohol Ether, PPG-10-Lanolin Alkohol Ether und PPG-30-Lanolin Alkohol Ether, alle Fa. Amerchol), Mono- und Oligoglyceride des Lanolins, von Lanolinalkoholen und Lanolinsäuren (z. B. Glyceryl Lanolat, Fa. Brooks; Neocerit, Fa. Beiersdorf),
f) Sterole aus Tallöl sowie deren ethoxylierte und/oder propoxylierte Derivate (z. B. PEG-5-Tallöl Sterol Ether),
g) Glycyrrhezitinsäure (Fa. Ichimaru Pharmacos) und Derivate (z. B. Glycyrrhetinyl Stearate; Fa. Maruzen oder Fa. Ichimaru Pharmacos),
h) Steroid-Vitamine und deren synthetische Analoga (z. B. Vitamine D₂, D₃ und D₄, Calcipotriol),
i) Steroidhormone natürlichen und synthetischen Ursprungs (z. B. Androgene, Östrogene, Gestagene, Corticoide, Mineralocorticoide oder Ecdyson),
j) natürliche und synthetische Cardenolide (z. B. Digitoxin, Dogoxin, Digoxygenin, Gitoxygenin, Strophanthin und Strophanthidin),
k) natürliche und synthetische Bufadienolide (z. B. Scillaren A, Scillarenin und Bufotalin),
l) Sapogenine und Steroid-Sapogenine (z. B. Amyrine, Oleanolsäure, Digitonin, Gitogenin, Tigogenin und Diosgenin),
m) Steroid-Alkaloide pflanzlichen und tierischen Ursprungs (.z. B. Tomatidin, Solanin, Solanidin, Conessin, Batrachotoxin und Homobatrachotoxin) sowie
n) die in folgender Aufstellung genannten, im Handel erhältlichen kosmetischen Rohstoffe:

| Substanz | Handelsname | Lieferant |
|---|---|---|
| Beta-Sitosterol | | |
| Beta-Sitosterylacetat | Sitostearyl Complex | Variati |
| Cholecalciferol | Epiderm-Complex O | Cosmetochem |
| Cholesterol | Cholesterol USP/NF | Croda |
| Cholesteryl Hydroxystearat | Estemol CHS | Nissin Oil Mills |
| | Salacos HS | Nissin Oil Mills |
| Cholesteryl Isostearat | IS-CE | Kao Corp. |
| Choleth-20 | Nikkol Aquasome EC-5 | Nikko |
| Choleth-24 | Fancol CH-24 | Fanning |
| Dihydrocholesterol | | |
| Dihydrocholesteryl Octyldecanoat | Nikkol DCIS | Nikko |
| Dihydrocholeth-15 | Nikkol DHC-15 | Nikko |
| Dihydrocholeth-20 | Nikkol DHC-20 | Nikko |
| Dihydrocholeth-30 | Nikkol DHC-30 | Nikko |
| Dihydrolanosterol | Isocholesterol EX | Nikko |
| Dihydrophytosteryl Octyldecanoat | Nikkol DPIS | Nikko |
| Disodium Sitostereth-14 | | |
| Sulfosuccinat | Rewoderm SPS | Rewo Chem. |
| Ergocalciferol | | |
| Glyceryl Lanolat | Ivarlan 3360 | Brooks |
| Glyceryllanolinate | Neocerit | Beiersdorf |
| Glycyrrhecitinsäure | 18β-Glycyrrhecitinic acid | Ichimaru Pharmacos |
| Glycyrrhetinyl Stearat | Grhetinol-O | Maruzen |
| Laneth-5 | Polychol-5 | Croda |
| Laneth-10 | Polychol-10 | Croda |
| Laneth-15 | Polychol-15 | Croda |
| Laneth-20 | Polychol-20 | Croda |
| Laneth-25 | Soluan 25 | Amerchol |
| Laneth-50 | Sterol LN 50 | Auschem |
| Laneth-10 Acetat | Lipolan 98 | Lipo |
| Lanosterol | Lanosterol | Croda |
| | Isocholesterol EX | Nikko |
| PEG-5-Lanolat | Agnosol 5 | Croda |
| PEG-10-Lanolat | Sklirate 10 | Croda |
| PEG-20-Lanolat | Sklirate 20 | Croda |
| PEG-10-Lanolin | Nikkol TW-10 | Nikko |
| PEG-20-Lanolin | Solan 20 | Croda |
| PEG-30-Lanolin | Nikkol TW-30 | Nikko |
| PEG-40-Lanolin | Solan 40 | Croda |
| PEG-75-Lanolin | Aqualose L75 | Westbrook Lanolin |
| PEG-100-Lanolin | Aqualose L100 | Westbrook Lanolin |
| PEG-150-Lanolin | Solan X | Croda |
| PPG-2 Lanolin Alcohol Ether | Soluan PB-2 | Amerchol |
| PPG-5-Lanolin Alcohol Ether | Soluan PB-5 | Amerchol |
| PPG-10-Lanolin Alcohol Ether | Soluan PB-10 | Amerchol |
| PPG-20-Lanolin Alcohol Ether | Soluan PB-20 | Amerchol |
| PPG-30-Lanolin Alcohol Ether | Soluan PB-30 | Amerchol |
| PPG-5-Lanolin Wax | Propoxyol 1695 | Henkel |
| PPG-12-PEG-50 Lanolin | Lanexol AWS | Croda |
| PPG-12-PEG-65 Lanolin Oil | Fluilan AWS | Croda |
| PPG-40-PEG-60 Lanolin Oil | Aqualose LL 100 | Westbrook Lanolin |
| Stearyl Glycyrrhetinate | Co-Grhetinol | Maruzen |
| | ST-Glycyrrhetinate | Ichimaru Ph. |

• Kohlenhydrate und Kohlenhydrat-Derivate:
Vorteilhafte antimikrobielle Wirkstoffe im Sinne der vorliegenden Erfindung sind Zucker und substituierte Zucker oder Zuckerreste enthaltende Verbindungen. Zu den Zuckern zählen insbesondere auch jeweils die Desoxy- und Didesoxy-Formen, beispielsweise die im Folgenden genannten Wirkstoffe, wobei die Liste der genannten Substanzen selbstverständlich nicht limitierend sein soll:
1. Monosaccharide:
   Vorteilhafte Monosaccharide im Sinne der vorliegenden Erfindung sind z. B. Tetrosen, Pentosen, Hexosen und Heptosen. Bevorzugt werden Pentosen und Hexosen. Die Ringstrukturen umfassen Furanosen und Pyranosen, umfaßt sind sowohl D- als auch L-Isomere, ebenso wie α- und β-Anomere. Geeignet sind auch die Desoxy- und Didesoxy-Formen.
2. Disaccharide:
   Vorteilhafte Disaccharide im Sinne der vorliegenden Erfindung sind z. B. die durch binäre Verknüpfungen obiger Monosaccharide gebildeten Disaccharide. Die Verknüpfung kann als α- oder β-glycosidische Bindung zwischen den beiden Untereinheiten erfolgen. Saccharose, Maltose, Lactobiose werden bevorzugt. Ebenso geeignet sind N-Acetyl-Galactosamin- und N-Acetyl-Glucosamin-Derivate sowie Silalinsäure-substituierte Derivate.
3. Oligosaccharide (natürlichen und synthetischen Urpsrungs):
   Vorteilhafte Oligosaccharide im Sinne der vorliegenden Erfindung bestehen aus mehreren, z. B. 2 bis 7 Zuckereinheiten, vorzugsweise der unter Mono- und Disaccharide beschriebenen Zucker, insbesondere aus 2 bis 5 Einheiten in den bekannten, durch Kondensation entstandenen Bindungsformen und wie vorstehend genannt. Besonders bevorzugte Oligosaccharide sind neben den Disacchariden die Tri- und Tetrasaccharide. Ebenso geeignet sind N-Acetyl-Galactosamin- und N-Acetyl-Glucosamin-Derivate sowie Silalinsäure-substituierte Derivate.
4. Aminozucker:
   Vorteilhafte Mono-, Di- und Oligosaccharide im Sinne der vorliegenden Erfindung sind, insbesondere wie vorstehend beschrieben, solche, welche eine oder mehrere Aminogruppen tragen, die acyliert, insbesondere acetyliert sein können. Bevorzugt werden Ribosylamin, N-Acetylglucosamin und N-Galactosylamin sowie Sialinsäure-substituierte Derivate.
5. Zuckerester:
   Vorteilhaft im Sinne der vorliegenden Erfindung sind ferner Zuckerester von organischen oder anorganischen Säuren, beispielsweise Zuckerphosphate, Zuckerester mit Carbonsäuren oder sulfatierte Zucker, insbesondere Ester der vorstehend beschriebenen Zucker.
6. Zuckerester anorganischer Säuren:
   Bevorzugt im Sinne der vorliegenden Erfindung sind weiterhin Zuckerester der Phosphorsäure, wie z. B. Glucose-1-phosphat; Fructose-1-phosphat, Glucose-6-phosphat und/oder Mannose-6-phosphat.
7. Zuckerester organischer Säuren:
   Bevorzugt im Sinne der vorliegenden Erfindung sind ferner Ester aus Zuckern und Carbonsäuren, wie Ester von Zuckern mit Carbonsäuren der Kettenlänge C₁ bis C₂₄, zum Beispiel Cetearylglucosid (Fa. Seppic: Montanol 68), Caprylyl/Caprylglucosid (Fa. Seppic: Oramix CG-110), Decylglucosid (Fa. Seppic: Oramix NS-10), Sucroselaurat und -myristat (Fa. Ryoto Sugar), Sucrose Cocoat (Fa. Croda), ferner insbesondere Zuckeracetate, bevorzugt der vorstehenden Zucker.
8. Zuckerether:
   Bevorzugt sind ferner Zuckerether aus Zuckern, insbesondere den vorstehenden Zucker, und ein- und mehrwertigen Alkoholen der Kettenlänge C₁ bis C₂₄, z. B. Plantaren^{R} 1200 (Fa. Henkel) oder Plantaren^{R} 2000 (Fa. Henkel).
   Weiterhin vorteilhaft sind z. B. die Umsetzungsprodukte von Zuckern mit Ethylenoxid und/oder Propylenoxid, vorzugsweise mit den vorstehenden Zuckern. Besonders vorteilhaft sind Ethoxylierungs- bzw. Propoxylierungs-Grade von einer bis 40 Ethereinheiten.
9. Glykolipide (natürlichen und synthetischen Ursprunges)
   Bevorzugte Glykolipide sind Glycosylglycerole, Monoacylglycosylglycerole und Diacylglycosylglycerole sowie Glykosphingolipide, insbesondere Ceramide, Cerebroside, Ganglioside und Sulfatide (z. B. Globotriaosylceramid; Globotetraosyceramid, Globopentaosylceramid; Forssmann-Glycolipid und Analoga); Glycocerebroside (z. B. Glucocerebroside, Galactocerebroside, Lactocerebroside), N-Alkanoylcerebroside (z. B. N-Palmitoylcerebrosid, N-Stearoylcerebrosid), N-Alkenoylcerebroside (z. B. N-Nervonoylcerebrosid, N-Oleoylcerebrosid), Cerebrosidsulfat sowie Ganglioside (z. B. Typen 1 bis 5, Asialogangliosid GM1, Asialogangliosid GM2, Asialogangliosid GM3, Monosialogangliosid GM1, Monosialogangliosid GM2, Monosialogangliosid GM3, Disialogangliosid GD1a, Disialogangliosid GD1b, Disialogangliosid GD2, Disialogangliosid GD3, Trisialogangliosid GT1b, Tetrasialogangliosid GQ1b) sowie Tenside auf Chitinbasis.
10. Polysaccharide (natürlichen und synthetischen Ursprunges)
   Vorteilhaft sind verzweigte und/oder unverzweigte Polysaccharide (Homopolysaccharide und Heteropolysaccharide), jeweils insbesondere mit den oben beschriebenen Zuckern. Bevorzugte Polysaccharide sind Stärke, Glykogen, Cellulose, Dextran, Tunicin, Inulin, Chitin, insbesondere Chitosane, Chitinhydrolysate, Alginsäure und Alginate, Pflanzengumme, Körperschleime, Pektine, Mannane, Galactane, Xylane, Araban, Polyosen, Chondroitinsulfate, Heparin, Hyaluronsäure und Glycosaminoglykane, Hemicellulosen, substituierte Cellulose und substituierte Stärke, insbesondere jeweils die hydroxyalkylsubstituierten Polysaccharide. Besonders geeignet sind ferner Amylose, Amylopektin, Xanthan, α-, β- und γ-Dextrin. Die Polysaccharide können z. B. aus 4 bis 1.000.000, insbesondere aus 10 bis 100.000 Monosacchariden bestehen. Vorzugsweise werden jeweils solche Kettenlängen gewählt, die gewährleisten, daß der Wirkstoff in der jeweiligen Zubereitung löslich oder in sie einzuarbeiten ist.
   Die unter a) bis j) genannten Wirkstoffe können sowohl einzeln als auch in beliebigen Mischungen untereinander vorliegen. Es ist insbesondere vorteilhaft, Monosaccharide und Oligosaccharide miteinander zu kombinieren, wobei jeweils ein Saccharid, aber auch zwei oder drei oder mehrere Zucker gewählt werden können. Zusammen mit den vorstehend genannten Zuckern oder deren Kombinationen können vorteilhaft ein Polysaccharid oder auch mehrere Polysaccharide verwendet werden.
   Fucose, Galactose, Sialinsäure, N-Acetyl-Galactosamin und N-Acetyl-Glucosamin werden besonders bevorzugt.
   Bevorzugt werden antimikrobielle Wirkstoffkombinationen mit mindestens drei antimikrobiellen Wirkstoffen, ausgewählt aus der Gruppe enthaltend:
   Aldopentosen und Ketopentosen und Aldohexosen und Ketohexosen und Aldoheptosen und Ketoheptosen.
   Die genannten Zucker können insbesondere auch in ihrer Desoxy- und Didesoxy-Form und insbesondere auch in der Form der erfindungsgemäßen Derivate vorliegen. Dies gilt auch für die folgenden bevorzugten Kombinationen.
   Besonders bevorzugt sind ferner antimikrobielle Kombinationen, insbesondere antimikrobielle Kombinationen von mindestens drei Wirkstoffen, die mindestens einen Desoxy- oder Didesoxy-Zucker oder mindestens ein Desoxy- oder Didesoxy-Zucker-Derivat oder mindestens ein Disaccharid oder mindestens ein Trisaccarid oder mindestens ein Tetrasaccharid enthalten, wobei diese auch jeweils in der Form der erfindungsgemäßen Derivate oder auch in der Desoxyoder Didesoxy-Form vorliegen können.
   Weiterhin bevorzugt sind antimikrobielle Kombinationen, insbesondere antimikrobielle Kombinationen von mindestens drei Wirkstoffen, die Fuctose, Galactose, Sialinsäure, N-Acetyl-Galactosamin und N-Acetyl-Glucosamin enthalten, wobei diese auch jeweils in der Form der erfindungsgemäßen Derivate vorliegen können.
   Besonders bevorzugt sind ferner die folgenden antimikrobiellen Wirkstoffkombinationen a) bis f):
   a) Fucose, Raffinose und Galactose
   b) Glucose-6-phosphat, Mannose-6-phosphat und Mannose
   c) Raffinose, N-Acetyl-glucosamin, und Fucose
   d) Mannose, Rhamnose und Fucose
   e) Galactose, N-Acetyl-glucosamin und Fucose
   f) Mannose, Raffinose und Galactose.
   Bevorzugt werden auch die jeweiligen antimikrobiellen Einzelkomponenten der Kombinationen und die mit jeweils zwei Komponenten zu bildenden antimikrobiellen Zweier-Kombinationen aus den drei Wirkstoffen jeweils einer Dreier-Kombination.
   Vorteilhaft können auch jeweils mit einem Zucker oder mehreren Zuckern aus der Gruppe der Monosaccharide und/oder der Oligosaccharide ein oder mehrere Zucker aus der Gruppe der Zuckerphosphate und/oder der Aminozucker und Acetylaminozucker kombiniert werden.
   In den antimikrobiellen Kombinationen können die Wirkstoffe z. B. mit gleichen Gewichtsmengen oder auch z. B. im Gewichtsverhältnis von 1 : 1000 bis 1000 : 1, vorzugsweise 1 : 10 bis 10 : 1 verwendet werden, jeweils bezogen auf eine oder mehrere andere Komponenten.

Besonders vorteilhafte Repellent-Wirkstoffe im Sinne der vorliegenden Erfindung sind die obengenannten Wirkstoffe N,N-Diethyl-3-methylbenzamid, 3-(N-n-Butyl-N-acetyl-amino)-propionsäureethylester und Dimethylphthalat.

Erfindungsgemäß bevorzugt sind ferner die im folgenden aufgelisteten Repellent-Wirkstoffe:

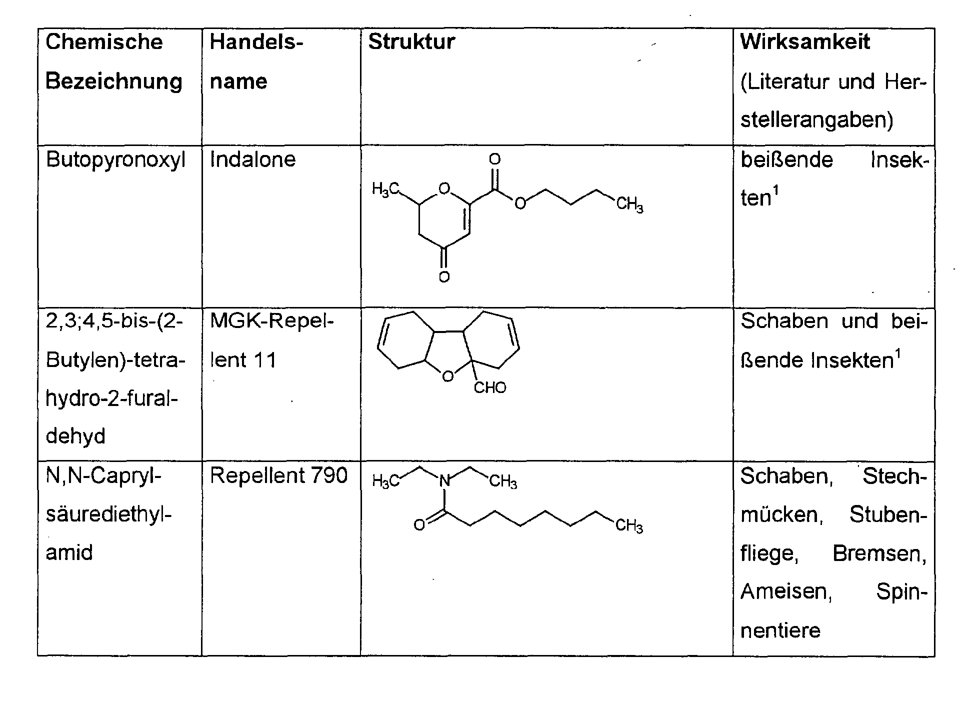

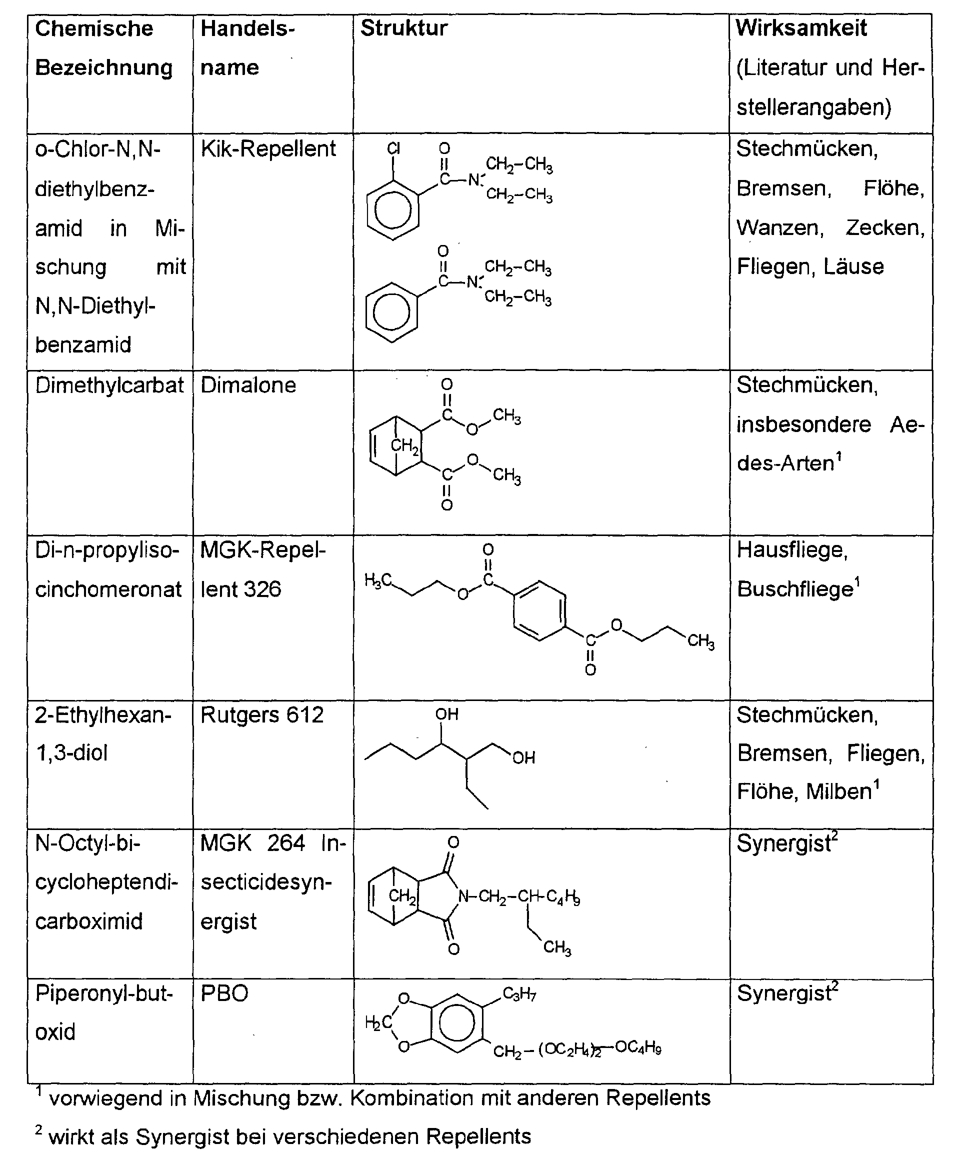

Erfindungsgemäß werden die Wirkstoffkombinationen bevorzugt in kosmetischen oder dermatologischen Zusammensetzungen in einem Gehalt von 0,005 bis 70,0 Gew.-% eingesetzt, insbesondere 0,01 bis 50,0 Gew.-%, bezogen auf das Gesamtgewicht der Zusammensetzung bevorzugt sind. Vorteilhaft enthalten die Zusammensetzungen 0,02 bis 10,0 Gew.-%, besonders bevorzugt 0,02 bis 5,0 Gew.-% an den erfindungsgemäßen Wirkstoffkombinationen, ganz besonders vorteilhaft 0,5 bis 3,0 Gew.-%, jeweils bezogen auf das Gesamtgewicht der Zusammensetzung.

Es ist bevorzugt im Sinne der vorliegenden Erfindung, das Gewichtsverhältnis von Repellent zum antimikrobiellen Wirkstoff (jeweils eine oder mehrere Verbindungen) aus dem Bereich von 1 : 1000 bis 1000 : 1, vorzugsweise wie 1 : 10 bis 10 : 1 zu wählen.

Es ist auch von Vorteil, anstatt reiner erfindungsgemäßer Wirkstoffe solche Stoffe zu verwenden, welche sich ihrerseits durch einen Gehalt an erfindungsgemäßen Verbindungen auszeichen.

Die erfindungsgemäßen Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z. B. Nagellacke, Nagellackentferner, Nagelbalsame), Stifte und dergleichen.

Es ist auch möglich und gegebenenfalls vorteilhaft, die erfindungsgemäßen Wirkstoffkombinationen mit anderen Wirkstoffen zu kombinieren, beispielsweise mit anderen antimycotisch bzw. antiviral wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,5 zu wählen.

Die erfindungsgemäßen kosmetischen und/oder dermatologischen Formulierungen können wie üblich zusammengesetzt sein und zum Schutz der Haut und/oder der Haare vor Belästigung durch blutsaugende oder beißende Insekten und andere Parasiten dienen. Sie können aber beispielsweise auch in pflegenden kosmetischen Formulierungen oder Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Die erfindungsgemäßen kosmetischen Zubereitungen können kosmetische Hilfsstoffe enthalten, wie sie üblicherweise in solchen Zubereitungen verwendet werden, z. B. Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß vorteilhaft sind Emulsionen mit einem Gehalt an Wirkstoffkombinationen im Sinne der vorliegenden Erfindung. Sie enthalten z. B. die genannten Fette, Öle, Wachse und anderen Fettkörper, sowie Wasser und einen Emulgator, wie er üblicherweise für einen solchen Typ der Formulierung verwendet wird.

Gele enthaltend Wirkstoffkombinationen gemäß der Erfindung enthalten üblicherweise ferner Alkohole niedriger C-Zahl, z. B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthaltend Wirkstoffkombinationen gemäß der Erfindung enthalten z. B. natürliche oder synthetische Wachse, Fettalkohole oder Fettsäureester. Bevorzugt werden Lippenpflegestifte sowie desodorierende Stifte ("Deo-Sticks").

Als Treibmittel für aus Aerosolbehältem versprühbare kosmetische oder dermatologische Zubereitungen enthaltend erfindungsgemäße Wirkstoffkombinationen sind die üblichen bekannten leichtflüchtigen, verflüssigten Treibmittel, z. B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwassertoffe (FCKW).

Bevorzugt können Zubereitungen enthaltend erfindungsgemäße Wirkstoffkombinationen zudem Substanzen enthalten, die UV-Strahlung im UVB-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen. Vorteilhaft enthalten diese zusätzlich mindestens einen UVA-Filter und/oder mindestens einen UVB-Filter und/oder mindestens ein anorganisches Pigment.

Kosmetische Zubereitungen enthaltend Wirkstoffkombinationen gemäß der Erfindung zum Schutze der Haut vor UV-Strahlen können in verschiedenen Formen vorliegen, wie sie z. B. üblicherweise für diesen Typ von Zubereitungen eingesetzt werden. So können sie z. B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, eine Pickering-Emulsion, eine PIT-Emulsion, einen festen Stift oder auch ein Aerosol darstellen.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-B-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz und/oder ein Silikonderivat als weitere Phase enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Dioctylbutylamidotriazon (INCI: Dioctylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVI-NUL® T 150 vertrieben wird.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-Filtersubstanzen können öllöslich oder wasserlöslich sein.
Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Vorteilhafte wasserlösliche Filtersubstanzen sind z. B.:
Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Die Liste der genannten UV-Filter, die im Sinne der vorliegenden Erfindung eingesetzt werden können, soll selbstverständlich nicht limitierend sein.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die folgenden Beispiele sollen die Verkörperungen der vorliegenden Erfindung verdeutlichen. Die Angaben beziehen sich stets auf Gewichts-%, sofern nicht andere Angaben gemacht werden.

### Beispiele:

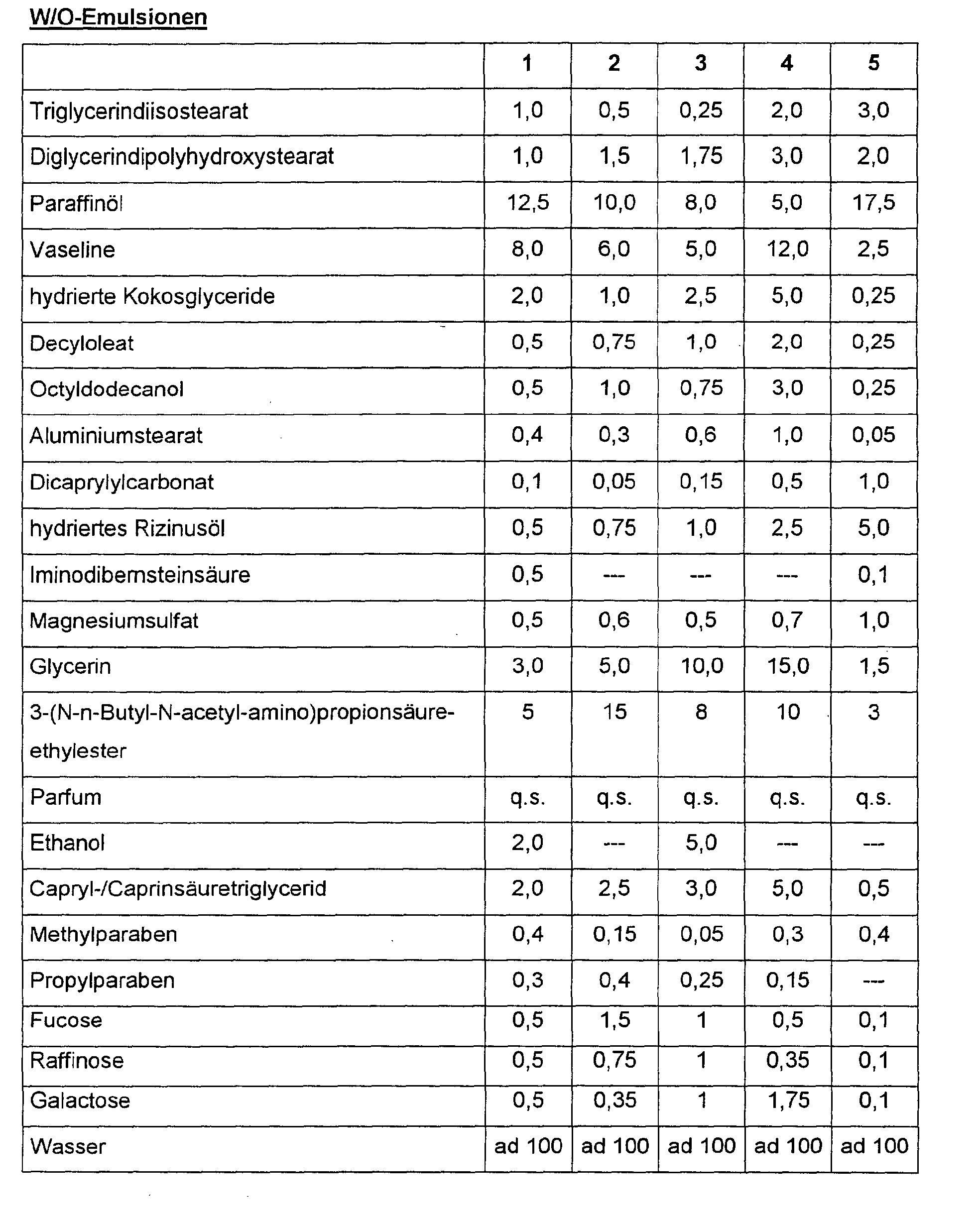

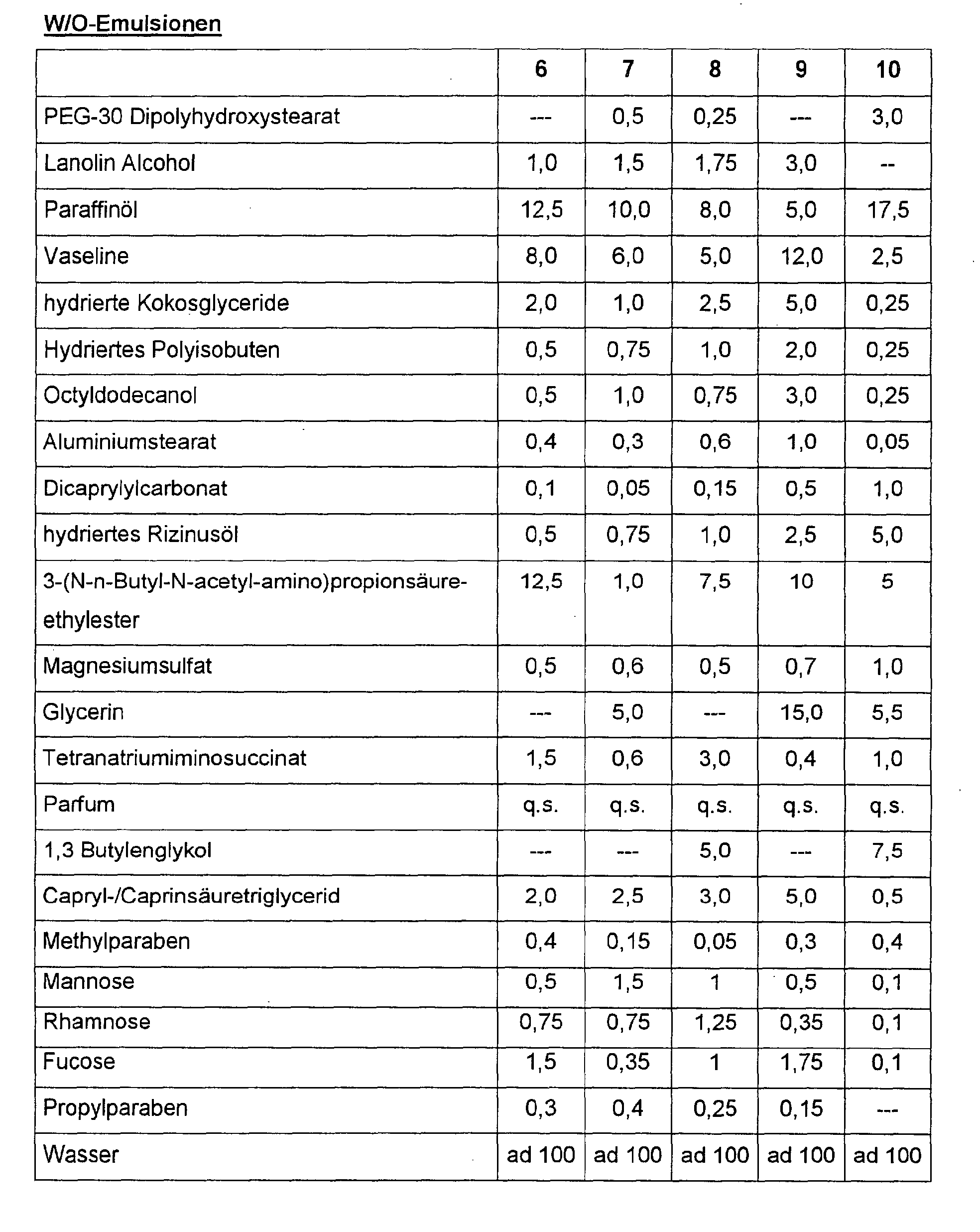

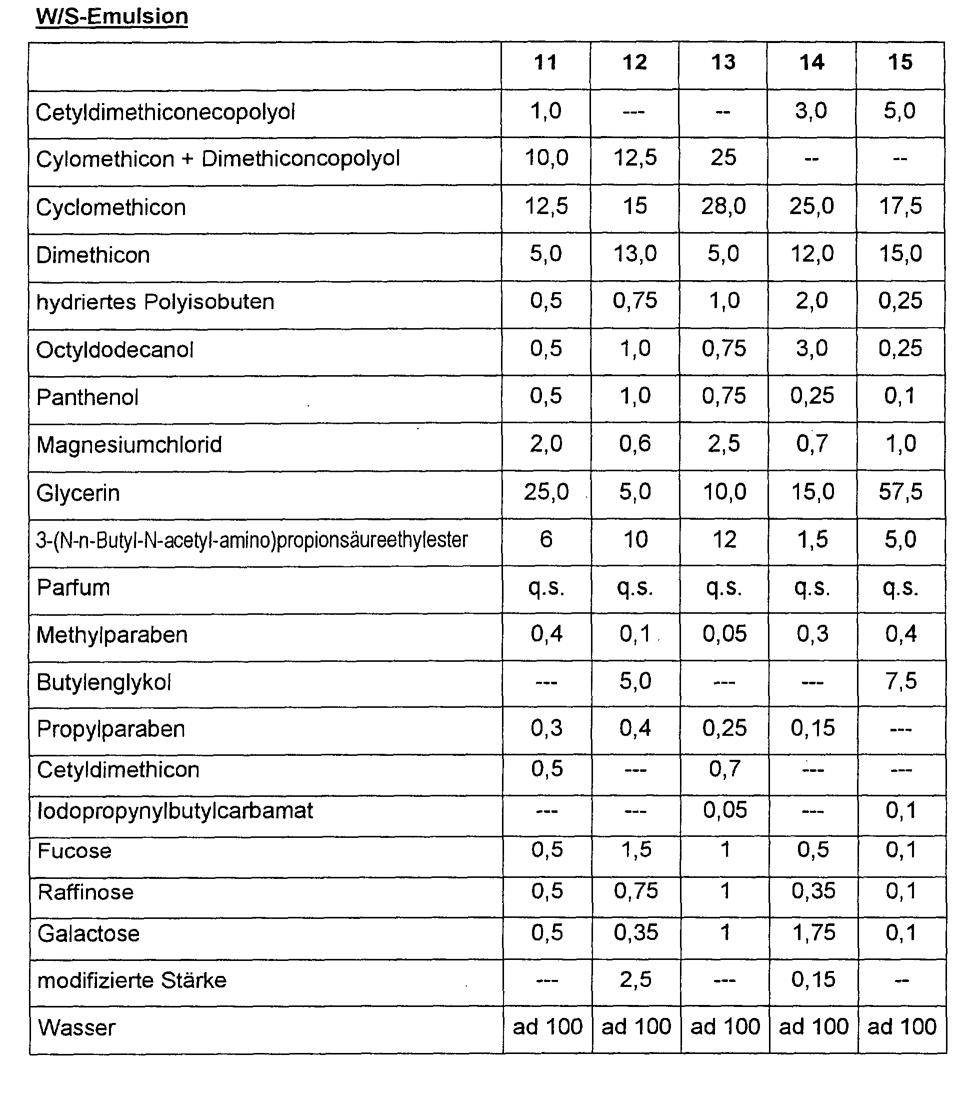

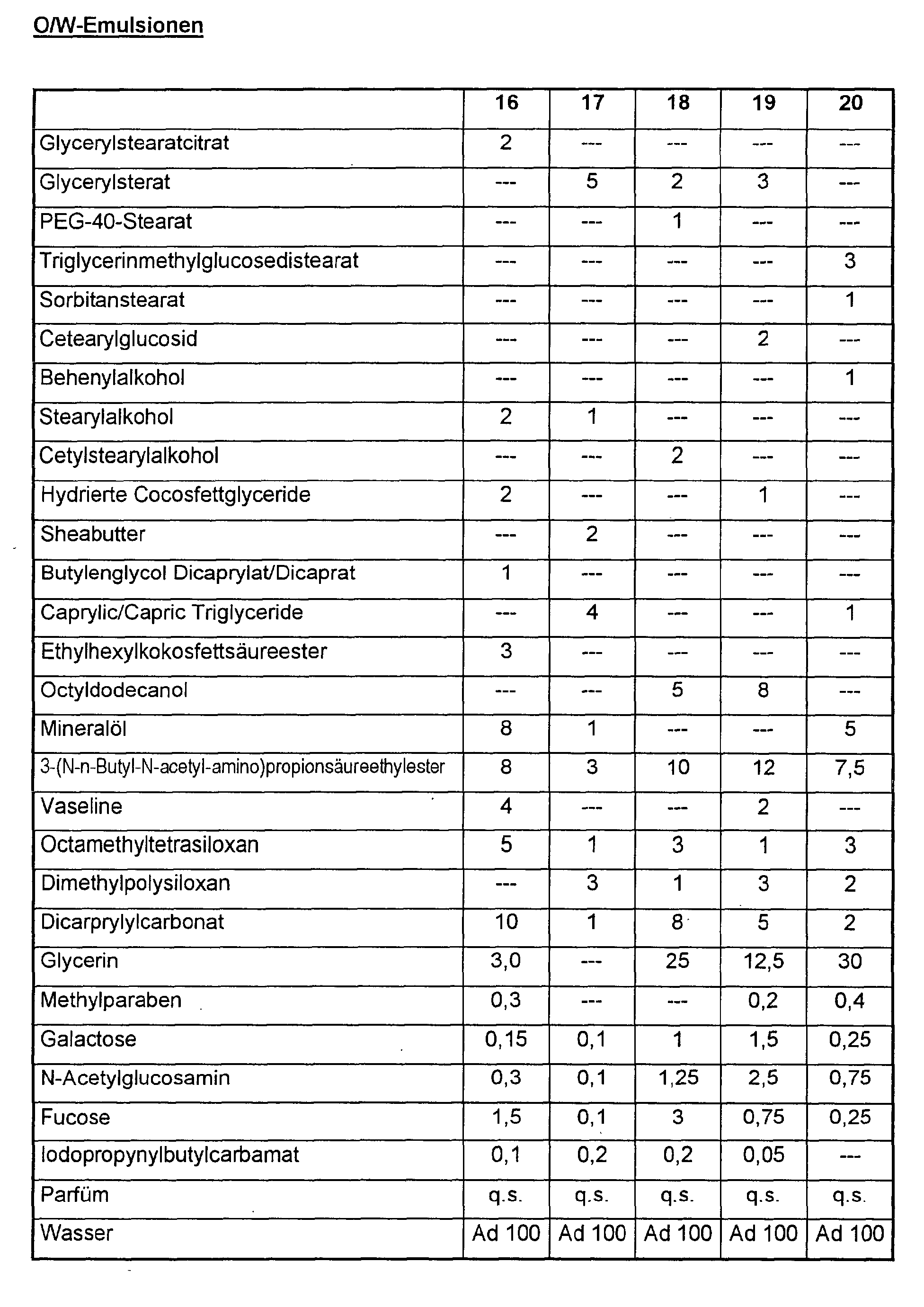

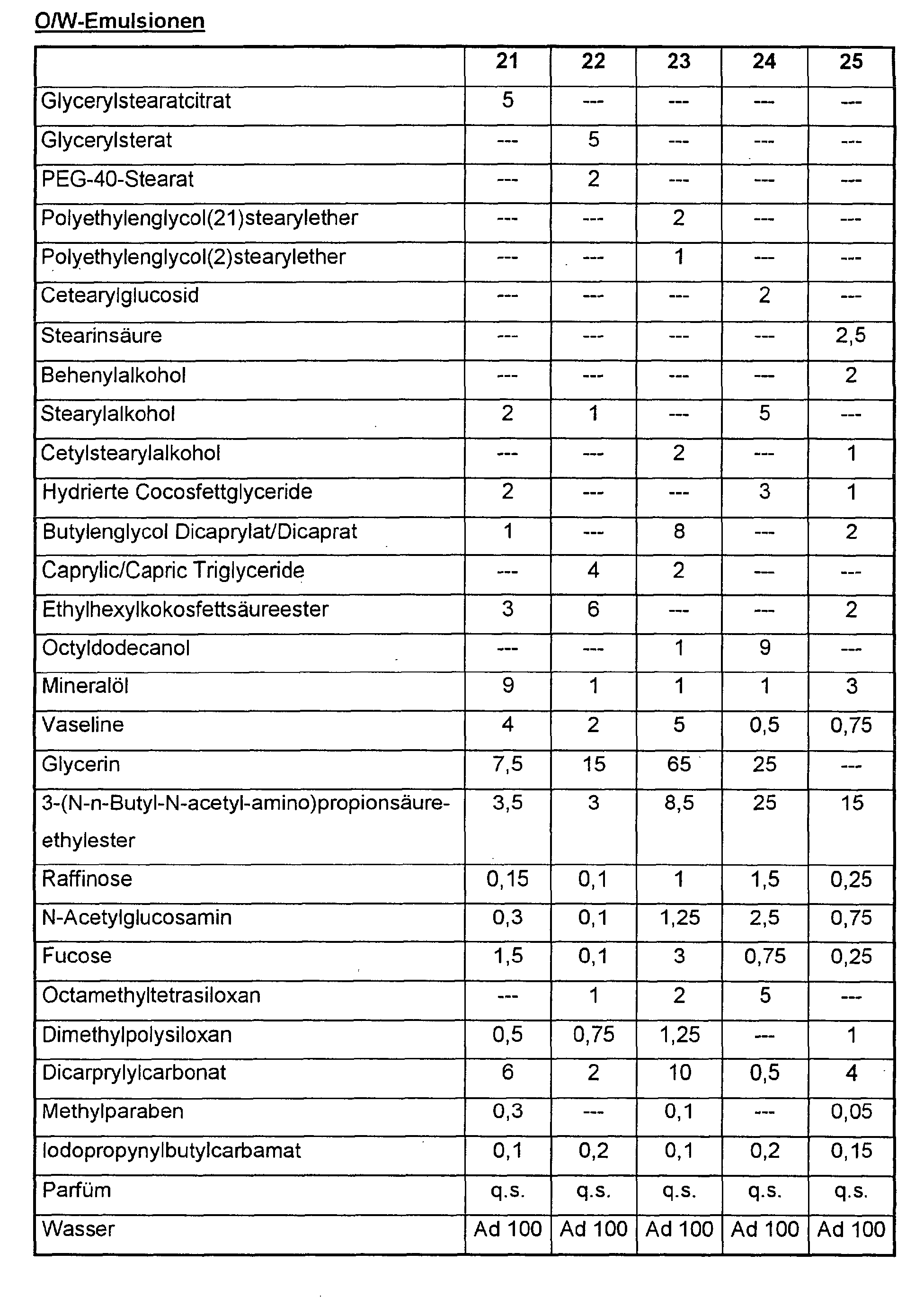

## Patentansprüche

1. Wirkstoffkombinationen zur Abwehr und/oder Vertreibung von stechenden oder beißenden Insekten und/oder anderen Parasiten und/oder Lästlingen aus mindestens einem Repellent-Wirkstoff und mindestens einem antimikrobiellen Wirkstoff.

2. Kosmetische und dermatologische Formulierungen, **dadurch gekennzeichnet, daß** sie Wirkstoffkombinationen aus
(a) mindestens einem Repellent-Wirkstoff und
(b) mindestens einem antimikrobiellen Wirkstoff
enthalten.

3. Kosmetische und dermatologische Formulierungen, **dadurch gekennzeichnet, daß** sie synergistische Wirkstoffkombinationen von
(a) mindestens einem Repellent-Wirkstoff und
(b) mindestens einem antimikrobiellen Wirkstoff
enthalten,
wobei die Schutzleistung dieser Zubereitungen gegen Lästlinge höher ist als die gleicher Zubereitungen, welche keine Substanzen gemäß (b) enthalten.

4. Zubereitung nach einem der Ansprüche 2 oder 3, **dadurch gekennzeichnet, daß** der oder die Repellent-Wirkstoff(e) aus der Gruppe der Aminopropionate gewählt wird/werden.

5. Zubereitung nach einem der vorhergehenden Ansprüche, **dadurch gekennzeichnet, daß** der oder die antimikrobielle(n) Wirkstoff(e) aus der Gruppe der Kohlenhydrate und Kohlenhydrat-Derivate gewählt wird/werden.

6. Zubereitung nach einem der vorhergehenden Ansprüche, **dadurch gekennzeichnet, daß** die antimikrobiellen Wirkstoffe in Form von Wirksystemen bestehend aus mindestens drei Wirkstoffen vorliegen.

7. Zubereitung nach Anspruch 6, **dadurch gekennzeichnet, daß** das Wirksystem eine der folgenden Kombinationen a) bis f) darstellt:
(a) Fucose, Raffinose und Galactose
(b) Glucose-6-phosphat, Mannose-6-phosphat und Mannose
(c) Raffinose, N-Acetyl-glucosamin, und Fucose
(d) Mannose, Rhamnose und Fucose
(e) Galactose, N-Acetyl-glucosamin und Fucose
(f) Mannose, Raffinose und Galactose.

8. Zubereitung nach einem der vorhergehenden Ansprüche, **dadurch gekennzeichnet, daß** das Verhältnis von Repellent zum antimikrobiellen Wirkstoff (jeweils eine oder mehrere Verbindungen) aus dem Bereich von 1 : 10 bis 10 : 1 gewählt wird.

9. Verwendung von mindestens einem antimikrobiellen Wirkstoff zur Erhöhung der Wirksamkeit von Repellent-Wirkstoffen.
